Europäisches Patentamt

**European Patent Office**

**Office européen des brevets**

(19)

(11) Publication number: **0 141 627**
A2

(12) # EUROPEAN PATENT APPLICATION

(21) Application number: **84307363.6**

(22) Date of filing: **25.10.84**

(51) Int. Cl.⁴: **B 29 C 59/14,** C 07 K 17/08,
G 01 N 33/545, C 12 Q 1/00

(30) Priority: **25.10.83 JP 199856/83**

(43) Date of publication of application: **15.05.85**
**Bulletin 85/20**

(84) Designated Contracting States: **DE FR NL**

(71) Applicant: **SUSUMU INDUSTRIAL CO., LTD., No. 14,**
**Umawawashi-cho Kamitoba Minami-ku, Kyoto-shi**
**Kyoto-fu (JP)**
Applicant: **SHIONOGI & CO., LTD., 12,**
**Dosho-machi 3-chome Higashi-ku, Osaka 541 (JP)**

(72) Inventor: **Horiguchi, Kazuo c/o Susumu Industrial Co.,**
**Ltd., No. 14, Umamawashi-cho Kamitoba Minami-ku,**
**Kyoto-shi Kyoto-fu (JP)**
Inventor: **Uo, Junko, 79, Nakabayashi-cho Shugakuin**
**Sakyo-ku, Kyoto-shi Kyoto-fu (JP)**

(74) Representative: **Bizley, Richard Edward et al, BOULT,**
**WADE & TENNANT 27 Furnival Street, London EC4A 1PQ**
**(GB)**

(54) **Carrier film for immobilizing protein and its production, and protein immobilized thereby and the use thereof.**

(57)    A carrier film for immobilizing protein on which non-specific adsorption of protein is suppressed comprises a polymeric film at least one surface of which is incinerated using a low temperature plasma of oxygen or oxygen plus an inert gas.

EP 0 141 627 A2

## CARRIER FILM FOR IMMOBILIZING PROTEIN
## AND ITS PRODUCTION, AND PROTEIN
## IMMOBILIZED THEREBY AND THE USE  THEREOF

The present invention relates to a carrier film for immobilizing protein and the production thereof.

Clinical analysis is carried out to analyze chemicals contained in human fluids and tissues to aid the prevention, diagnosis and/or therapy of disease.  In clinical analysis, it is required that a microamount of a sample can be quickly and easily analyzed with accuracy.

Since enzymes have various characteristics which are not found in chemical reagents such as high specificity and the ability to accelerate reactions under mild reaction conditions they are increasingly used in clinical analysis. Since free enzymes are, however, expensive and unstable, they are usually immobilized on polymeric carriers for use in immobilized form.

As a method for the clinical analysis of minor biomaterials, immunoassay has been employed.  In this  method, an antigen or antibody which participates in the immunoreaction  is usually immobilized on the carrier and thus used.

When protein (e.g. enzymes, antigens and antibodies) is immobilized on carriers, the following factors should be considered:

1.    The density of protein immobilized on the carrier should be sufficiently high.

2.    In the antigen-antibody reaction, non-specific adsorption should occur less frequently than adsorption due to the reaction.

3.    When the carrier is in the form of a film and is used in an enzyme-electrode method, the permeating rate through the film for reaction products between the enzyme and a substrate should be large.

Hitherto, such carrier films have been prepared mainly by a casting method (cf. S. Kato, M. Aizawa & S Susuki, J. Membr. Sci., 3, 29 (1978) and N. Weliky & H. H. Weetall, Immunochemistry, 2, 293 (1965), etc.). The casting of a polymer requires, however, high skill and its reproducibility is not good.

The present invention aims to provide a carrier film for immobilizing protein which can usually be easily prepared at low cost. The invention also aims to provide a carrier film which suppresses non-specific adsorption of protein.

Thus, according to one aspect of the present invention there is provided a carrier film for immobilizing protein

- 3 -

0141627

comprising a polymeric film at least one surface of which has been incinerated by a low temperature plasma of oxygen or oxygen plus an inert gas.

According to another aspect of the present invention, there is provided a method for producing a carrier film for immobilizing protein comprising treating a polymeric film with a low temperature plasma of oxygen or oxygen plus an inert gas to incinerate at least one surface of the polymeric film.

The polymeric carrier film of the invention may be solid or porous. When it is used in an enzyme-electrode method, it must be porous. In case of a porous film, its pore size is preferably at least 250 Å.

The thickness of the film is not critical in the present invention. Preferably, it is less than 100 micrometers before incineration. When it is thicker than 100 micrometers, it takes a longer time thoroughly to incinerate the pore walls of the porous film. Since a film which is too thin is difficult to handle, it is preferably at least 25 micrometers in thickness. The thickness of the film after incineration is preferably at least about 15 micrometers to produce a self-supporting film. When the film is supported by a separate supporting member, the thickness may, however, be thinner than 15 micrometers.

As the polymeric film to be used in the invention, any one that comprises carbon atoms, hydrogen atoms and optionally oxygen atoms and has sufficient mechanical

- 4 -                                    0141627

strength to stand handling can be used.  Preferred examples of the polymer are, e.g., polyolefins (e.g. polypropylene, etc.) and polycarbonates.

The carrier film of the invention may be produced by incinerating the polymeric film using a low temperature plasma of oxygen or oxygen plus or inert gas by means of conventional plasma generating equipment.  Thus, at least one surface of the polymeric film may be incinerated by placing it on an electrode of the plasma generating equipment and discharging between the electrodes in a reduced pressure atmosphere of oxygen or oxygen plus an inert gas to generate gaseous plasma.

In the plasma treatment according to the invention, pure oxygen is preferred.  However, oxygen may be diluted with an inert gas (eg. helium, argon, nitrogen, etc.).

The pressure during discharging is usually from 0.15 to 1 Torr.  When it is less than 0.15 Torr, higher voltage is required for discharging.  On the other hand, when it is higher than 1 Torr it is difficult to discharge.

The current density is from 0.05 to 0.5 mA/cm$^2$.

The discharging time varies with other conditions and can be easily determined by those skilled in the art, for example, by referring to the Examples given below.  In the case of porous film, in order to incinerate sufficiently

- 5 -

0141627

the pore walls it is treated for such a period that the film thickness is decreased by at least 20 %.

In the present invention, the polymeric film is incinerated on one surface, preferably on both surfaces to produce a high quality carrier film.

Since aldehyde groups are formed on the incinerated surface(s) of the polymeric film, the completion of incineration may be ascertained by dyeing the surface with Schiff's reagent.

Specific examples of proteins which may be immobilized on the carrier film of the invention are enzymes, antigens and antibodies. Protein can be immobilized on the carrier film by a method conventional per se, for example, by immersing the carrier film in a solution of protein.

The carrier film of the invention treated by a low temperature plasma has improved wettability towards water and exhibits so-called "spreading" wetting. Thus, its ability to make uniform contact with an electrode for measurement and its affinity to a substrate solution are greatly improved and, accordingly, enzyme reactions proceed smoothly. Since the incineration of the polymeric film substantially reduces the film thickness and the film surface is roughened thus increasing the surface area, the density of immobilized protein is increased resulting in an improvement in measuring sensitivity. In addition, the reduction in film thickness accelerates the permeation of a material produced by an enzyme reaction through the carrier film, which further improves measuring sensitivity.

One of the significant characteristics of the carrier film of the invention incinerated by a low temperature plasma is that non-specific adsorption of protein, particularly enzyme-labelled antigens and the like which are used in immunoassay, is greatly suppressed. For this reason, background "noise" in measurement is lowered and the accuracy of measurement is improved.

The present invention will now be described further by way of the following Examples giving specific embodiments.    In the accompanying drawings :-

Fig. 1 is a graph showing the results of Example 1, and

Fig. 2 is a graph showing the results of Example 2.

Example 1

A parallel flat plate type plasma reactor made of glass (300 mm in diameter and 500 mm in length) was used.

A polypropylene film ("Juragurad" (trade name) #3401 manufactured by Polyplastic Kabushiki Kaisha) was placed on an aluminium electrode of the reactor.  After evacuating the reactor to 0.001 Torr or less, oxygen gas was injected in the reactor and, then, the reactor was discharged with conditions of 100 mA (0.14mA/cm$^2$) and 350 V while evacuating the reactor to maintain 0.3 Torr.

The discharge was continued for 30 minutes during which the film was cut off every 5 minutes.

When incinerating both surfaces, each surface of the film was discharged for 15 minutes whilst turning it over every 5 minutes.

The sample film cut off at each interval was divided into five pieces each having the same size.

The samples were immersed in distilled water and installed on a hydrogen peroxide electrode and then immersed in a buffer (PBS). Direct current of +0.65 V was applied to a platinum electrode at 25°C with stirring at 300 rpm. After stabilization, current flow through 0.5 ml of a 0.03 % solution of hydrogen peroxide was measured.

The results in the cases of both one surface treatment and both surface treatment are shown in Table 1.

Table 1

| Treating time (min.) | Output current[*1] through $H_2O_2$ (x100 nA) | |
|---|---|---|
| | One surface | Both surfaces |
| 0 | 46.0 | 45.2 |
| 5 | 49.2 | 56.8 |
| 10 | 52.2 | 64.2 |
| 15 | 53.2 | 70.8 |
| 20 | 58.0 | ——— |
| 25 | 61.2 | ——— |
| 30 | 69.0 | 99.8 |

Note: *1) Average of five measurements for one
      surface treatment and of four measurements
      for both surface treatment since one film
      was broken.

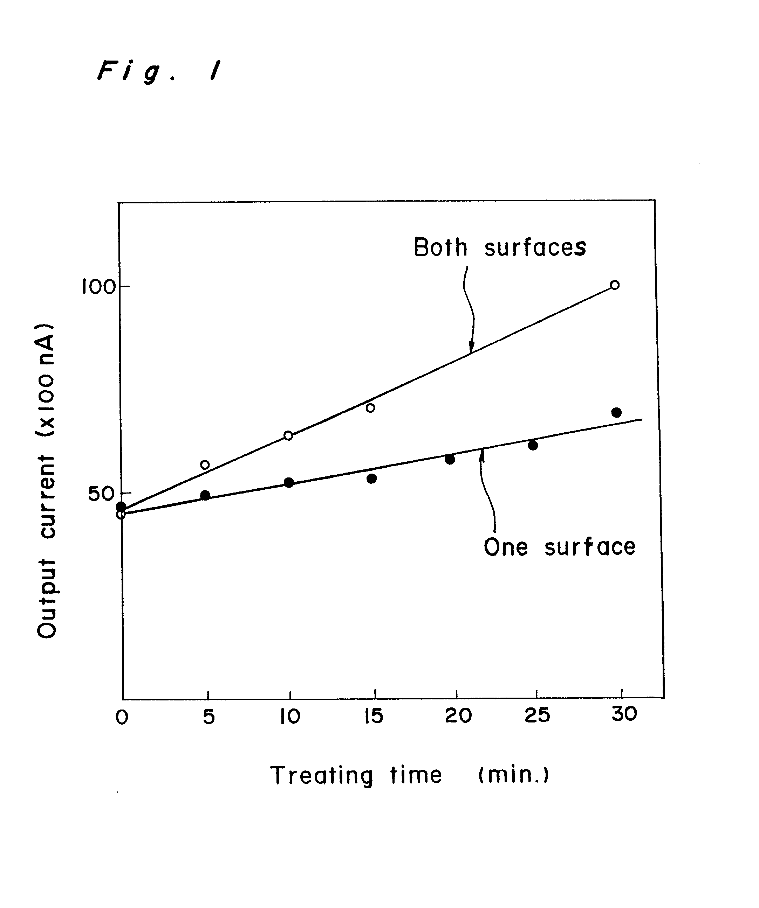

These results are plotted in the graph of Fig. 1.

Weight decrease of the films, both surfaces of
which were discharged for 30 minutes, was calculated as
follows:

The polypropylene film before incineration was dip
washed in distilled water, air dried and weighed. After
plasma treatment, it was again weighed. Weight decrease (%)
and then the thickness after incineration was calculated
from the thickness before incineration (25 micrometers).
The results are as follows:

|  | Before | After | Weight decrease |
|---|---|---|---|
| Sample No. 1: | 0.24775 g | 0.15400 g | 0.09375 g (37.8 %) |
| Sample No. 2: | 0.21250 g | 0.14610 g | 0.06640 g (31.2 %) |

Thickness of the film after incineration

Sample No. 1: 25 - (25 x 0.387) = ca.15.5 micrometers
Sample No. 2: 25 - (25 x 0.312) = ca.17.2 micrometers

Example 2 and Comparative Example 1

In the same manner as in Example 1, a polypropylene film ("Juragurad" (trade name) #3401 manufactured by Polyplastic Kabushiki Kaisha. 20 x 20 cm square) was plasma treated for 15 minutes. From the weight decrease by incineration, it was found that the film thickness was reduced from 25 micrometers before incineration to 18 micrometers. The gloss on the surface disappeared. When water was dropped on the surface, it spread over the whole surface area and was absorbed by the film.

The thus obtained incinerated film and untreated film were immersed in a solution of insulin labelled with glucose oxydase at 25°C for 90 minutes and washed with a buffer.

Each film was installed on a hydrogen peroxide electrode and its glucose oxydase (GOD) activity was measured.

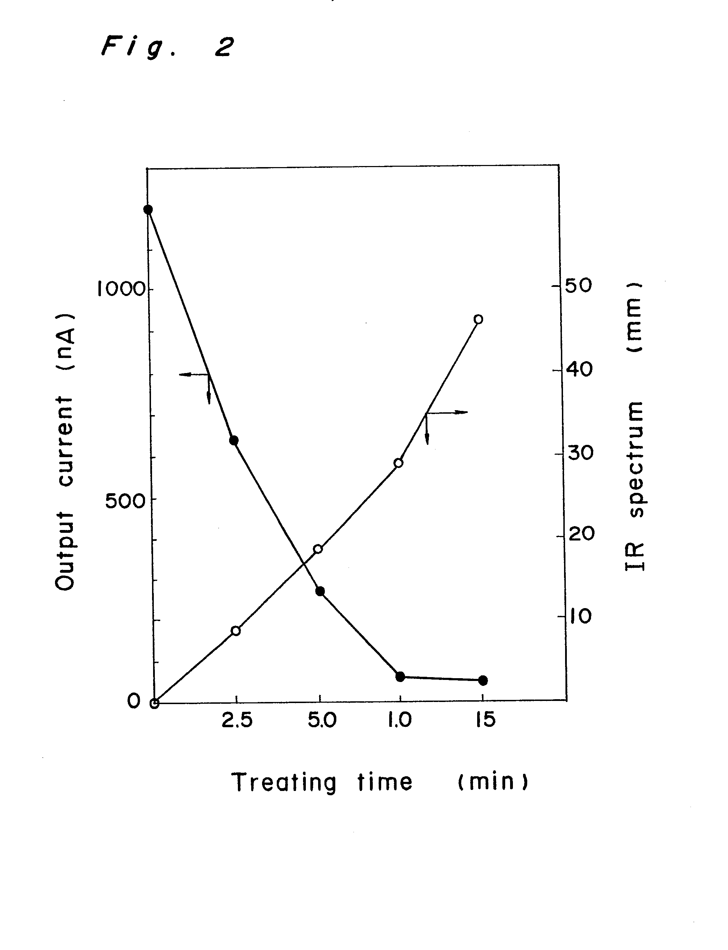

Output current based on the GOD activity for the film incinerated for 15 minutes according to ·the present invention was only 50 nA while that of the untreated polypropylene film was 1,200 nA. Output currents for films incinerated for various periods shorter than 15 minutes were measured. All the results are shown in Table 2 and plotted in the graph of Fig. 2.

In these Examples, output current due to adsorption based on the GOD activity was measured as follows:

The sample film was treated in the solution of insulin labelled with GOD at 25°C for 90 minutes and washed with a buffer (PBS) four times. Then, it was installed on a hydrogen oxide electrode and immersed in 30 ml of PBS. Direct current of +0.65 V was applied to the platinum electrode and stabilized. Thereafter, 20 mg of glucose was added and the current flowing was measured.

Table 2

| Treating time (both surfaces) (min.) | Height of IR spectrum peak at 1,710 cm$^{-1}$ (mm) | Output current based on GOD activity (nA) |
|---|---|---|
| Untreated | 0 | 1,200 |
| 2.5 | 7.5 | 640 |
| 5 | 17.5 | 265 |
| 10 | 29.0 | 60 |
| 15 | 46.0 | 50 |

The surfaces of the films treated according to the present invention were dyed purplish red with Schiff's reagent. This indicates the presence of aldehyde groups on the surfaces of the films to which a primary amine can be bonded.

Example 3

A polypropylene film incinerated by a plasma of oxygen for 15 minutes was after-treated by stirring it in a 2.5 % aqueous solution of octamethylene diamine at 25°C for 90 minutes, washing with distilled water three times and, then, stirring in a 2.5 % aqueous solution of glutaraldehyde at 25°C for 90 minutes and thoroughly washing with distilled water.

On the thus after-treated film and the film obtained in Example 2 (15 minute treatment), human albumin labelled with GOD was immobilized by contacting the film with a solution thereof at 25°C for 90 minutes.

Each film on which human albumin was immobilized was installed on a hydrogen peroxide electrode, and the amount of the GOD-labelled human albumin bonded to the film was measured by way of the output current based on GOD activity. The output current for the after-treated film was 9,990 nA while that for the non-after-treated film was 22 nA.

The output current for the non-after-treated film was due to non-specific adsorption. The output current for the after-treated film was about 500 times as high as that for the non-after-treated film.

- 12 -                              0141627

CLAIMS :

1.      A carrier film for immobilizing protein comprising a polymeric film at least one surface of which has been incinerated using a low temperature plasma of oxygen or oxygen plus an inert gas.

2.      A carrier film according to claim 1, wherein the polymeric film is porous.

3.      A carrier film according to claim 2, wherein the pore size of the polymeric film is at least 250 Å.

4.      A carrier film according to any one of claims 1 to 3, wherein the polymeric film comprises carbon atoms, hydrogen atoms and optionally oxygen atoms.

5.      A carrier film according to claim 4, wherein the polymeric film is a polyolefin, e.g. polypropylene, or a polycarbonate film.

6.      A carrier film according to any one of claims 1 to 5, wherein the thickness of the polymeric film before incineration is from 25 to 100 micrometers and after incineration is at least 15 micrometers.

0141627

7.     A method for producing a carrier film for immobilizing protein comprising treating a polymeric film with a low temperature plasma of oxygen or oxygen plus an inert gas to incinerate at least one surface of the polymeric film.

8.     A method according to claim 7, wherein the plasma is a low temperature plasma of pure oxygen.

9.     A method according to claim 7 or claim 8, wherein the plasma treatment is carried out at a pressure of from 0.15 to 1 Torr.

10.     A method according to any one of claims 7 to 9, wherein the thickness of the polymeric film is reduced by at least 20 %.

11.     A method according to any one of claims 7 to 10 and further defined by the specific feature of any one of claims 2 to 5.

12.     A protein immobilized on a carrier film according to any one of claims 1 to 6.

13.     The use of a protein according to claim 12 in an analysis or assay technique.

Fig. 1

0141627

2/2

Fig. 2